# EUROPÄISCHE PATENTANMELDUNG

(11) **EP 1 798 552 A1**
(43) Veröffentlichungstag der Anmeldung: **20.06.2007**
(21) Anmeldenummer: 05027708.6
(22) Anmeldetag: 17.12.2005
(51) Int. Cl.: G01N 33/26

(54) **Sensorvorrichtung für Schmier- oder Hydrauliköle**

(71) Anmelder: ARGO-HYTOS GmbH, 76703 Kraichtal-Menzingen (DE)
(72) Erfinder: Mann, Wolfgang Dr., 76684 Östringen-Tiefenbach (DE); Meindorf, Thomas Dr., 76689 Karlsdorf Neuthard (DE)
(74) Vertreter: Hoeger, Stellrecht & Partner Patentanwälte

(57) **Zusammenfassung**

Die Erfindung betrifft eine Sensorvorrichtung (10; 40; 50; 80) zur Erfassung von mindestens einer Eigenschaft eines Schmier- oder Hydrauliköls mit zumindest einem Sensorelement (20; 70). Um die Sensorvorrichtung derart weiterzubilden, daß die Gefahr einer Belagbildung oder Verschmutzung der Oberfläche des Sensorelements (20; 70) vermindert werden kann, wird erfindungsgemäß vorgeschlagen, daß die Sensorvorrichtung (10; 40; 50; 80) ein Filterelement (23; 42; 65) umfaßt, wobei das mindestens eine Sensorelement (20; 70) auf der Reinölseite des Filterelements (23; 42; 65) angeordnet ist.

## Beschreibung

Die Erfindung betrifft eine Sensorvorrichtung zur Erfassung von mindestens einer Eigenschaft eines Schmier- oder Hydrauliköls mit zumindest einem Sensorelement.

Derartige Sensorvorrichtungen kommen beispielsweise zur Überwachung der Qualität von Schmier- oder Hydraulikölen zum Einsatz. Die Überwachung physikalischer oder chemischer Eigenschaften von Ölen gewinnt im Zusammenhang mit der Zustandsüberwachung von Anlagen und Maschinen zunehmend an Bedeutung. Es ist das Ziel, Maschinenschäden und Maschinenstillstandszeiten aufgrund von Verschleiß oder gealterten oder falschen Schmier- oder Hydraulikölen zu reduzieren oder sogar ganz zu vermeiden. Maschinenausfälle sind in vielen Fällen auf gealterte, falsche oder kontaminierte Schmier- und Hydrauliköle zurückzuführen.

Zur Überwachung der Ölqualität wird mindestens eine physikalische oder chemische Eigenschaft des Öls mittels eines Sensorelements erfaßt. Das Ausgangssignal des Sensorelements kann einer Auswerteelektronik zugeführt werden, die dann die vom Sensorelement erfaßte Eigenschaft im Hinblick auf die Qualität des Öles beurteilt. Das Sensorelement kontaktiert zumindest während des Betriebs der Sensorvorrichtung das zu überwachende Öl. Hierbei kann es zu Belagbildungen und Verschmutzungen auf der das Öl kontaktierenden Oberfläche des Sensorelements kommen. Derartige Belagbildungen und Verschmutzungen treten insbesondere dann auf, wenn die Sensoroberfläche gegenüber der Sensorumgebung auf einem anderen elektrischen Potential liegt, denn dadurch werden geladene Teilchen in Richtung auf die Sensoroberfläche beschleunigt. Auch ungeladene, aber polare Teilchen erfahren aufgrund der häufig starken inhomogenen elektrischen Felder, die in der unmittelbaren Umgebung des Sensorelements wirken, eine Beschleunigung in Richtung auf das Sensorelement. Derartige Belagbildungen und Verschmutzungen können die Funktion des Sensorelements beeinträchtigen. Dies gilt insbesondere für Sensorelemente mit mikrostrukturierten Oberflächen, wie sie beispielsweise bei kapazitiven Sensoren mit seitlich nebeneinander liegenden und in einander greifenden Kammelektroden zum Einsatz kommen können. Leitfähige Partikel, zum Beispiel Metallspäne oder Rußpartikel, können sich an den Elektroden ablagern und zu Kurzschlüssen führen.

Aufgabe der vorliegenden Erfindung ist es, eine Sensorvorrichtung der eingangs genannten Art derart weiterzubilden, daß die Gefahr einer Belagbildung oder Verschmutzung der Oberfläche des Sensorelements vermindert werden kann.

Diese Aufgabe wird bei einer Vorrichtung der gattungsgemäßen Art erfindungsgemäß dadurch gelöst, daß die Sensorvorrichtung ein Filterelement umfaßt, wobei das mindestens eine Sensorelement auf der Reinölseite des Filterelements angeordnet ist. Durch die Bereitstellung des Filterelements ist sichergestellt, daß zwar das zu untersuchende Öl an die Oberfläche des Sensorelements gelangen kann, daß aber Verunreinigungen vom Filterelement zurückgehalten werden. Die Gefahr einer Belagbildung oder Verschmutzung der Sensoroberfläche wird dadurch ganz erheblich gemindert.

Es kann vorgesehen sein, daß das Filterelement und das Sensorelement an einem gemeinsamen Träger gehalten sind, wobei das Filterelement mit dem Träger lösbar verbindbar ist. Dies gibt die Möglichkeit, das Filterelement auf einfache Weise auszutauschen, falls es nach längerem Gebrauch der Sensorvorrichtung verstopft ist.

Von Vorteil ist es, wenn das Filterelement das Sensorelement in Umfangsrichtung umgibt. Eine derartige Ausgestaltung schützt das Sensorelement nicht nur vor Verunreinigungen sondern auch vor mechanischen Beeinträchtigungen, da das Filterelement für das Sensorelement ein schützendes Gehäuse ausbildet.

Alternativ kann vorgesehen sein, daß die Sensorvorrichtung ein Gehäuse mit einem Öleinlaß und einem Ölauslaß aufweist, die über einen Strömungsweg miteinander verbunden sind, wobei das Filterelement im Strömungsweg angeordnet ist und das Sensorelement stromabwärts des Filterelements positioniert ist. Bei einer derartigen Ausgestaltung wird der mechanische Schutz des Sensorelements durch das Gehäuse der Sensorvorrichtung gewährleistet. Das Gehäuse kann von dem zu untersuchenden Öl durchströmt werden, wobei das Öl zunächst auf das Filterelement trifft, dieses durchströmt oder mittels Diffusionsvorgänge passiert und anschließend mit hohem Reinheitsgrad auf die Oberfläche des stromabwärts des Filterelements positionierten Sensorelements trifft.

Das Filterelement umfaßt ein Filtermaterial, das beispielsweise in Form einer öldurchlässigen Membran, eines Papierfilters und/oder eines Vlieses ausgestaltet sein kann.

Es kann vorgesehen sein, daß das Filterelement aus einem öldurchlässigen gesinterten Werkstoff hergestellt ist, beispielsweise in Form einer gesinterten Membran. Von besonderem Vorteil ist es, wenn ein metallischer Sinterwerkstoff zum Einsatz kommt, der vorzugsweise mit einem elektrischen Massepotential verbindbar ist, um statische Aufladungen zu vermeiden.

Von besonderem Vorteil ist es, wenn das Filterelement zumindest eine elektrisch leitfähige Schicht aufweist. Dadurch können elektrostatische Aufladungen des Filterelements durch Ölverunreinigungen verhindert werden.

Bei einer besonders bevorzugten Ausgestaltung weist das Filterelement zumindest ein elektrisch leitfähiges Gewebe auf. Hierbei kann es sich um ein Stützgewebe handeln, an dem ein Vlies oder ein sonstiges als Filtermaterial zum Einsatz kommendes öldurchlässiges Flachmaterial vorzugsweise flächig anliegt. Bevorzugt kommt ein Metallgewebe oder ein Gewebe mit Metall- und Kunststoffäden zum Einsatz. Derartige Gewebe zeichnen sich durch eine besonders hohe mechanische Belastbarkeit aus, insbesondere im Hinblick auf wechselnde Druckbelastungen.

Günstig ist es, wenn mindestens eine elektrisch leitfähige Schicht des Filterelements das Sensorelement käfigartig umgibt, denn dies gibt die Möglichkeit, mit Hilfe des Filterelements nicht nur Verunreinigungen des Öls von der sensitiven Oberfläche des Sensorelements zurückzuhalten, sondern zusätzlich kann mittels des Filterelements eine elektrische Abschirmung des Sensorelements erzielt werden nach Art eines Faradaykäfigs.

Bevorzugt ist zumindest eine elektrisch leitfähige Schicht des Filterelements mit einer elektrischen Spannung beaufschlagbar. Durch Beaufschlagung der elektrischen leitfähigen Schicht mit einer elektrischen Spannung können geladene Partikel besonders wirkungsvoll von der Oberfläche des Sensorelements zurückgehalten werden. Die elektrisch leitfähige Schicht bildet somit ein Elektrofilter aus, das geladene Partikel zumindest einer Polarität von der sensitiven Oberfläche des Sensorelements abschirmt.

Bei einer besonders bevorzugten Ausführungsform weist das Filterelement mehrere elektrisch leitfähige Schichten auf, die mit unterschiedlichen elektrischen Spannungen beaufschlagbar sind. Insbesondere können die elektrisch leitfähigen Schichten mit Wechselspannungen beaufschlagt werden. Es hat sich gezeigt, daß durch das Anlegen unterschiedlicher elektrischer Potentiale an mehrere elektrisch leitfähige Schichten des Filterelements eine besonders wirkungsvolle elektrische Abschirmung des Sensorelements erzielt werden kann, denn es können geladene Partikel beider Polaritäten von der Sensoroberfläche fern gehalten werden. Außerdem können geladene Partikel von der Sensoroberfläche abgezogen werden, so daß diese durch Anlegen von mindestens einem elektrischen Potential an die elektrisch leitfähige Schicht des Filterelements gereinigt werden kann.

Das Sensorelement kann beispielsweise eine Kondensatoranordnung aufweisen, an die eine hochfrequente Meßspannung angelegt werden kann, insbesondere zur Bestimmung der relativen Dielektrizitätskonstante und/oder der elektrischen Leitfähigkeit des zu untersuchenden Öles.

Von Vorteil ist es, wenn das Sensorelement potentialfrei geschaltet werden kann. Dies ermöglicht einen intermittierenden Betrieb des Sensorelementes, wobei dieses in den Außenbetriebszeiten dasselbe elektrische Potential aufweisen kann wie das umgebende Öl, indem es potentialfrei geschaltet wird. Eine Drift geladener Teilchen in Richtung auf die Sensoroberfläche in Zeiten, in denen die Sensorvorrichtung außer Betrieb ist, wird dadurch vermieden. Die Gefahr einer Belagbildung oder Verschmutzung der Sensoroberfläche wird dadurch zusätzlich reduziert.

Zur weiteren Verminderung einer Belagbildung oder Verschmutzung ist es von Vorteil, wenn das Sensorelement eine haftvermindernde Schutzschicht aufweist, beispielsweise eine Schutzschicht aus einem haftvermindernden Material und/oder mit einer haftvermindernden Oberflächenstruktur. Es kann zum Beispiel eine dünne, fest haftende und temperaturstabile fluorhaltige Schicht zum Einsatz kommen. Derartige Schichten weisen eine sehr geringe Oberflächenenergie auf, ein Anhaften von Ölverunreinigungen wird dadurch eher unwahrscheinlich. Die Dicke der Schutzschicht kann sehr gering gehalten werden, sie kann beispielsweise ca. 10 nm bis ca. 10 µm betragen, so daß die Funktion des Sensorelements zur Erfassung einer Eigenschaft des Öls nicht wesentlich beeinträchtigt wird.

Um über sehr lange Zeitintervalle eine Beeinträchtigung der Funktion des Sensorelements durch Belagbildung oder Verschmutzung zu verhindern, ist bei einer besonders bevorzugten Ausführungsform vorgesehen, daß die Sensorvorrichtung ein piezoelektrisch in mechanische Schwingungen versetzbares Schwingteil aufweist, das mit dem Sensorelement gekoppelt ist. Mittels der mechanischen Schwingungen können Ölverunreinigungen auf einfache Weise von der Oberfläche des Sensorelements abgetrennt werden.

Bevorzugt ist das Sensorelement an einer Trägerplatine festgelegt, die an dem Schwingteil gehalten ist. Als Schwingteil kann beispielsweise ein Quarzsubstrat zum Einsatz kommen, an das eine hochfrequente elektrische Spannung angelegt werden kann und das starr mit der Trägerplatine verbunden ist, an der das Sensorelement festgelegt ist.

Von Vorteil ist es, wenn dem Sensorelement ein mechanisches Abstreifelement zugeordnet ist, das relativ zum Sensorelement entlang von dessen sensitiver Oberfläche bewegbar ist. Mittels des Abstreifelements können auf mechanische Weise Beläge und Verunreinigungen von der empfindlichen Oberfläche des Sensorelements entfernt werden. Als Abstreifelement kann zum Beispiel eine Gummilippe oder eine Bürstenleiste zum Einsatz kommen.

Günstig ist es, wenn das Abstreifelement mit einem Aktor gekoppelt ist zum Bewegen des Abstreifelements entlang der sensitiven Oberfläche des Sensorelements. Als Aktor kann beispielsweise ein Elektromotor zum Einsatz kommen oder eine Magnetspule.

Die nachfolgende Beschreibung bevorzugter Ausführungsformen der Erfindung dient im Zusammenhang mit der Zeichnung der näheren Erläuterung. Es zeigen:
- Figur 1:: eine schematische, teilweise geschnittene Seitenansicht einer ersten Ausführungsform einer erfindungsgemäßen Sensorvorrichtung;
- Figur 2:: eine schematische Darstellung von Detail A aus Figur 1;
- Figur 3:: eine schematische Seitenansicht einer zweiten Ausführungsform einer erfindungsgemäßen Sensorvorrichtung;
- Figur 4:: eine schematische Schnittansicht einer dritten Ausführungsform einer erfindungsgemäßen Sensorvorrichtung;
- Figur 5:: eine schematische Vorderansicht eines Sensorelements der Sensorvorrichtung aus Figur 4 mit zugeordneter Reinigungseinrichtung;
- Figur 6:: eine schematische Seitenansicht des Sensorelements aus Figur 5 mit zugeordneter Reinigungseinrichtung und
- Figur 7:: eine schematische Schnittansicht einer vierten Ausführungsform einer erfindungsgemäßen Sensorvorrichtung.

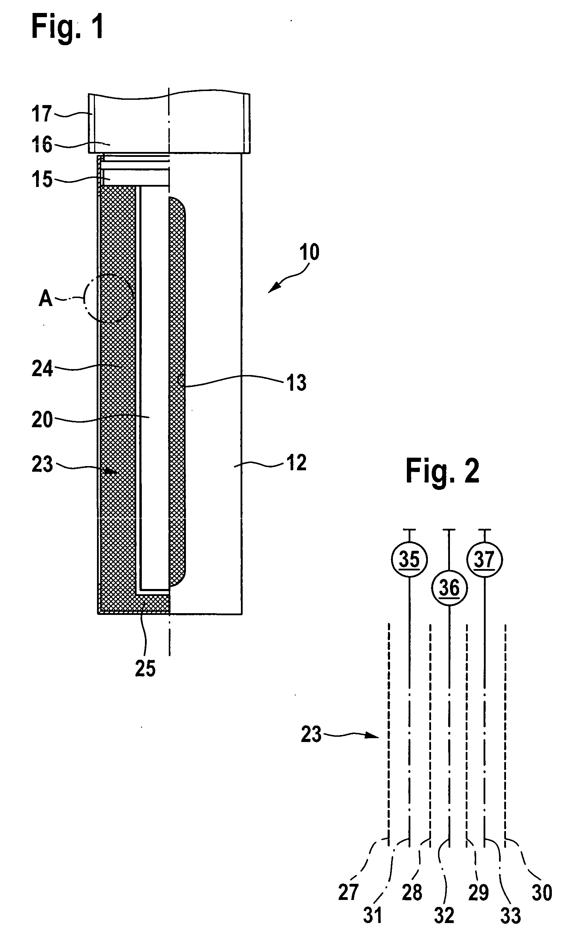

In Figur 1 ist schematisch, teilweise in Schnitt- und teilweise in Seitenansicht eine erste Ausführungsform einer erfindungsgemäßen Sensorvorrichtung 10 dargestellt mit einer zylinderförmigen Schutzhülse 12, die in Umfangsrichtung in gleichmäßigem Abstand zueinander vier spaltförmige, sich in Längsrichtung der Schutzhülse 12 erstreckende Durchgangsöffnungen 13 aufweist. Die Schutzhülse 12 ist auf ein Halteteil 15 aufgerastet, an das sich, der Schutzhülse 12 abgewandt, ein Gewindeteil 16 mit einem Außengewinde 17 anschließt. Mit Hilfe des Gewindeteils 16 kann die Sensorvorrichtung 10 in einen Behälter eingeschraubt werden, in dem sich zu untersuchendes Öl befindet.

In die Schutzhülse 12 taucht ein Sensorelement 20 ein, das am Halteteil 15 gehalten und von der Schutzhülse 12 abgedeckt ist. Sowohl das Halteteil 15 als auch das Gewindeteil 16 weisen eine in der Zeichnung nicht dargestellte axiale Durchgangsbohrung auf, durch die Anschlußkabel hindurchgeführt werden können, die das Sensorelement 20 kontaktieren. Letzteres kann beispielsweise als sogenannter SAW-Sensor (Surface Acoustic Wave-Sensor) oder auch als IDK-Sensor (Interdigitalkondensator-Sensor) ausgestaltet sein. Derartige Sensoren sind beispielsweise aus der WO 98/05953 bekannt. Mit ihrer Hilfe kann die Viskosität und/oder die Dielektrizitätskonstante des zu untersuchenden Öls erfaßt werden. Ein SAW-Sensor weist hierzu zwei in Dünnschichttechnik auf ein piezoelektrisches Material aufgebrachte Interdigitalwandler auf, um in einem sensitiven Oberflächenbereich des Sensorelements 20 eine Oberflächenwelle zu erzeugen, deren Dämpfung ein Maß für die Viskosität des angrenzenden Öls ist. Ein IDK-Sensor weist zwei ebenfalls in Dünnschichttechnik auf eine Trägerstruktur aufgebrachte Kammelektroden auf, die nebeneinander angeordnet sind und ineinander greifen und eine Kondensatoranordnung ausbilden. Mittels des IDK-Sensors kann beispielsweise die Leitfähigkeit des angrenzenden Öls erfaßt werden. Derartige Sensoren sind dem Fachmann bekannt, eine detaillierte Darstellung erübrigt sich daher.

Im Bereich zwischen der Schutzhülse 12 und dem Sensorelement 20 weist die Sensorvorrichtung 10 ein becherförmiges Filterelement 23 auf mit einem Filtermantel 24 und einem Filterboden 25. Das Filterelement 23 ist strömungsdicht am Halteteil 15 festgelegt und bildet eine öldurchlässige Hülle, die das Sensorelement 20 vor Schmutzpartikel schützt. In Figur 2 ist der Aufbau des Filterelements 23 schematisch dargestellt. Es weist mehrere Filtervliese 27, 28, 29, 30 auf, zwischen denen jeweils ein Metallgewebe 31, 32 bzw. 33 angeordnet ist. Die Metallgewebe 31, 32, 33 umgeben das Sensorelement 20 käfigartig und können jeweils an eine Spannungsquelle 35, 36 bzw. 37 angeschlossen werden. Über die Spannungsquellen 35, 36 und 37 können die Metallgewebe 31, 32 bzw. 33 mit unterschiedlichen und zeitlich wechselnden elektrischen Potentialen beaufschlagt werden, durch die elektrisch geladene Partikel des umgebenden Öls vom Sensorelement 20 zurückgehalten und außerdem eine Reinigung des Sensorelements 20 bewirkt werden kann.

Mittels des Sensorelements 20 können vorzugsweise mehrere physikalische und/oder chemische Eigenschaften des die Oberfläche des Sensorelements 20 kontaktierenden Öls erfaßt werden. Das Öl kann hierbei durch die Durchgangsöffnungen 13 und das Filterelement 23 hindurch strömen, wobei Partikel, die das Öl verunreinigen, von dem auf der Reinölseite des Filterelements 23 angeordneten Sensorelement 20 abgehalten werden, so daß eine Belagbildung und Verschmutzung der sensitiven Oberfläche des Sensorelements 20 sehr gering gehalten werden kann.

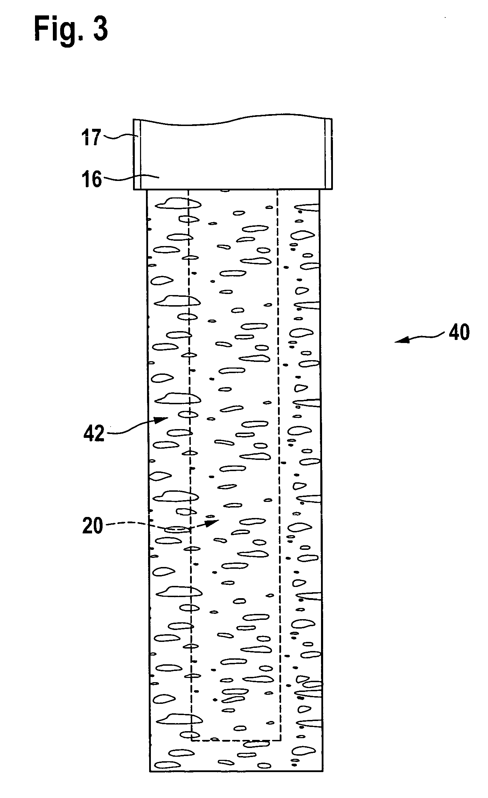

In Figur 3 ist eine zweite Ausführungsform einer erfindungsgemäßen Sensorvorrichtung dargestellt, die insgesamt mit dem Bezugszeichen 40 belegt ist. Diese ist ähnlich ausgestaltet wie die voranstehend unter Bezugnahme auf die Figuren 1 und 2 erläuterte Sensorvorrichtung 10, für identische Bauteile werden daher in Figur 3 dieselben Bezugszeichen verwendet wie in Figur 1 und zur Vermeidung von Wiederholungen wird bezüglich dieser Bauteile auf die voranstehenden Erläuterungen Bezug genommen.

Von der Sensorvorrichtung 10 unterscheidet sich die Sensorvorrichtung 40 durch den Einsatz eines gesinterten Filterelements 42, das eine öldurchlässige Hülle für das in das Filterelement 42 eintauchende Sensorelement 20 ausbildet. Das Filterelement 42 weist eine hohe Porosität auf und ist vorzugsweise aus Metall gefertigt. Dies gibt die Möglichkeit, an das Filterelement 42 ein elektrisches Potential anzulegen, um geladene Teilchen einer Polarität vom Sensorelement 20 zurückzuhalten, wie dies voranstehend bereits unter Bezugnahme auf die Metallgewebe 31, 32 und 33 der Sensorvorrichtung 10 erläutert wurde.

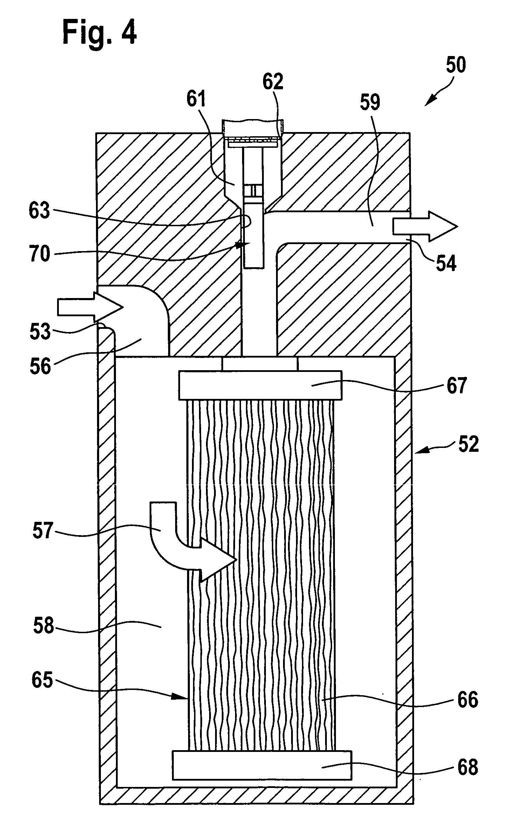

In den Figuren 4, 5 und 6 ist eine dritte Ausführungsform einer erfindungsgemäßen Sensorvorrichtung dargestellt, die insgesamt mit dem Bezugszeichen 50 belegt ist. Diese umfaßt ein Gehäuse 52 mit einem Öl-Einlaß 53 und einem Öl-Auslaß 54, die über einen Strömungsweg, nämlich einen Eingangskanal 56, einen sich in Strömungsrichtung 57 an diesen anschließenden Filterraum 58 und einen Ausgangskanal 59 miteinander in Strömungsverbindung stehen.

Im Filterraum 58 ist ein zylinderförmiges Filterelement 65 angeordnet mit einem mehrlagigen, beispielsweise aus Vlies und/oder Filterpapier gefertigten Filtermantel 66, der an seinen Stirnseiten oberseitig von einer oberen Endscheibe 67 und unterseitig von einer unteren Endscheibe 68 abgedeckt ist. Die obere Endscheibe 67 weist in üblicher Weise eine in der Zeichnung nicht dargestellte Durchgangsöffnung auf, so daß der vom Filtermantel 66 umgebene Innenraum des Filterelements 65 über die obere Endscheibe 67 mit dem Ausgangskanal 59 in Strömungsverbindung steht. Das Filterelement 65 kann von dem zu untersuchenden Öl radial von außen nach innen durchströmt werden.

Das Gehäuse 52 weist einen Sensorraum 61 auf, der über einen Sensoranschluß 62 von außen zugänglich ist und über einen Durchlaß 63 mit dem Ausgangskanal 59 in Strömungsverbindung steht. Der Sensorraum 61 nimmt ein Sensorelement 70 auf, das auf einer Trägerplatine 71 festgelegt ist, die ihrerseits an einer Ultraschallquelle in Form eines piezoelektrisch in mechanische Schwingungen versetzbaren Schwingteils 72 gehalten ist.

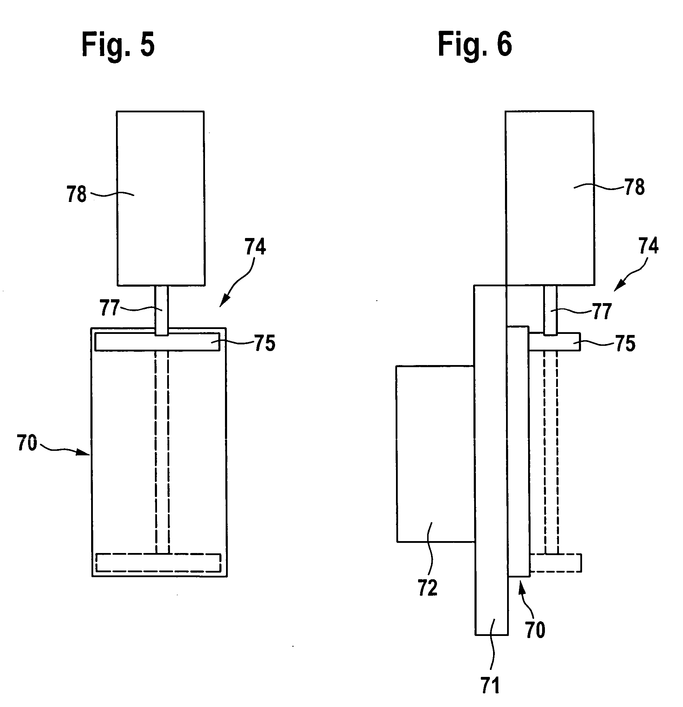

Der Sensorraum 61 nimmt außerdem eine Reinigungseinheit 74 auf mit einem Abstreifelement 75, beispielsweise einer Gummilippe oder einer Bürstenleiste, das auf der sensitiven Oberfläche des Sensorelements 20 aufliegt und über einen Schieber 77 mit einem Aktor 78, beispielsweise einer Magnetspule, gekoppelt ist. Mit Hilfe des Aktors 78 kann das Abstreifelement 75 entlang der Oberfläche des Sensorelements 70 hin und her verschoben werden. In den Figuren 5 und 6 ist das Abstreifelement 75 in durchgezogener Linie in einer zurückgezogenen Position und gestrichelt in einer ausgefahrenen Position dargestellt.

Die Sensorvorrichtung 50 kann in einen Ölkreislauf integriert werden. Über den Öl-Einlaß 53 kann das zu untersuchende Öl in das Gehäuse 52 eintreten. Es durchströmt anschließend das Filterelement 65, und auf der Reinölseite des Filterelementes 65, nämlich stromabwärts des Filterelements 65, trifft das zu untersuchende Öl auf das Sensorelement 70, das über den Durchlaß 63 ausgehend vom Sensorraum 61 in den Ausgangskanal 59 eintaucht und zumindest eine physikalische oder chemische Eigenschaft des Öls erfaßt. Ölverunreinigungen werden größtenteils vom Filterelement 65 zurückgehalten. Ein sich im Laufe der Zeit auf der Oberfläche des Sensorelements 70 dennoch ausbildender Schmutzbelag kann mittels der Reinigungseinheit 74 und des Schwingteils 72 entfernt werden, indem das Sensorelement 70 zusammen mit der Trägerplatine 71 vom Schwingteil 72 in mechanische Schwingungen versetzt wird und das Abstreifelement 75 mittels des Aktors 78 entlang der sensitiven Oberfläche des Sensorelements 70 hin und her vorschoben wird zur Reinigung der Sensoroberfläche.

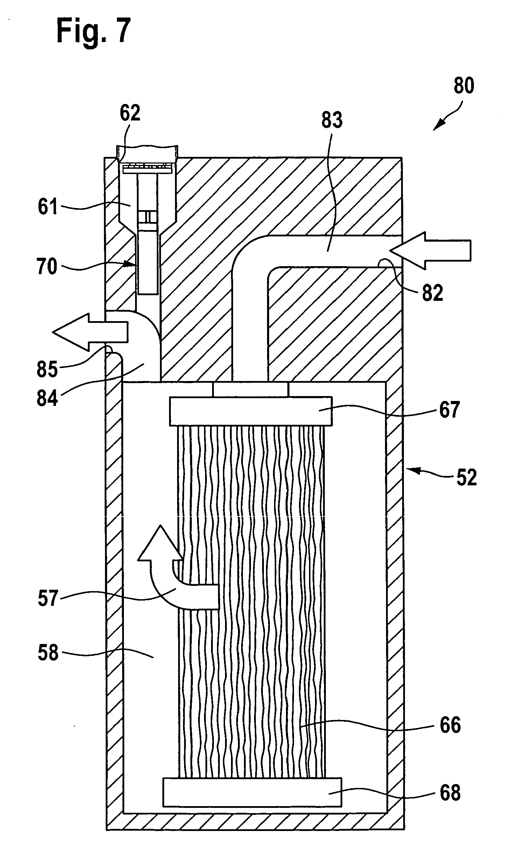

In Figur 7 ist eine weitere alternative Ausführungsform einer erfindungsgemäßen Sensorvorrichtung dargestellt, die insgesamt mit dem Bezugszeichen 80 belegt ist. Diese ist weitgehend identisch ausgebildet wie die voranstehend unter Bezugnahme auf die Figuren 4, 5 und 6 erläuterte Sensorvorrichtung 50. Für identische Bauteile werden daher in Figur 7 dieselben Bezugszeichen verwendet wie in den Figuren 4, 5 und 6. Bezüglich dieser Bauteile wird zur Vermeidung von Wiederholungen auf die voranstehenden Erläuterungen bezug genommen. Von der Sensorvorrichtung 50 unterscheidet sich die Sensorvorrichtung 80 dadurch, daß die Strömungsrichtung des zu untersuchenden Öles umgedreht ist, indem das zu untersuchende Öl zunächst über einen Öl-Einlaß 82, einen Eingangskanal 83 und die obere Endscheibe 67 in den Innenraum des Filterelements 65 eintritt und anschließend das Filterelement 65 radial von innen nach außen durchströmt. Über einen Ausgangskanal 84 und einen Öl-Auslaß 85 kann das gefilterte Öl aus dem Gehäuse 52 der Sensorvorrichtung 80 heraustreten.

Auch bei der Sensorvorrichtung 80 kommt ein Sensorelement 70 mit einer Trägerplatine 71 und einem Schwingteil 72 zum Einsatz sowie eine Reinigungseinheit 74 mit einem Abstreifelement 75, einem Schieber 77 und einem Aktor 78. Das Sensorelement 70 der Sensorvorrichtung 80 ist wie bei sämtlichen voranstehenden Ausführungsformen auf der Reinölseite des Filterelements 75 angeordnet und daher vor Verunreinigungen des zu untersuchenden Öls geschützt. Ein sich im Laufe der Zeit dennoch auf der Oberfläche des Sensorelements 70 ablagernder Belag kann mittels der Reinigungseinheit 74 und des Schwingteils 72 auf einfache Weise entfernt werden, ohne daß es hierzu erforderlich ist, das Sensorelement 70 auszutauschen.

## Patentansprüche

1. Sensorvorrichtung zur Erfassung von mindestens einer Eigenschaft eines Schmier- oder Hydrauliköls mit zumindest einem Sensorelement (20; 70), **dadurch gekennzeichnet, daß** die Sensorvorrichtung (10; 40; 50; 80) ein Filterelement (23; 42; 65) umfaßt, wobei das mindestens eine Sensorelement (20; 70) auf der Reinölseite des Filterelements (23; 42; 65) angeordnet ist.

2. Sensorvorrichtung nach Anspruch 1, **dadurch gekennzeichnet, daß** das Filterelement (23; 42) das Sensorelement (20) in Umfangsrichtung umgibt.

3. Sensorvorrichtung nach Anspruch 1, **dadurch gekennzeichnet, daß** die Sensorvorrichtung (50; 80) ein Gehäuse (52) mit einem Öl-Einlaß (53; 82) und einem Öl-Auslaß (54; 85) aufweist, die über einen Strömungsweg (56, 58, 59; 83, 58, 84) miteinander verbunden sind, wobei das Filterelement (65) im Strömungsweg angeordnet ist und das Sensorelement (70) stromabwärts des Filterelements (65) positioniert ist.

4. Sensorvorrichtung nach einem der voranstehenden Ansprüche, **dadurch gekennzeichnet, daß** das Filterelement (42) aus einem öldurchlässigen gesinterten Werkstoff hergestellt ist.

5. Sensorvorrichtung nach einem der voranstehenden Ansprüche, **dadurch gekennzeichnet, daß** das Filterelement (23; 42) zumindest eine elektrisch leitfähige Schicht (31, 32, 33) aufweist.

6. Sensorvorrichtung nach Anspruch 5, **dadurch gekennzeichnet, daß** das Filterelement (23) zumindest ein elektrisch leitfähiges Gewebe (31, 32, 33) aufweist.

7. Sensorvorrichtung nach Anspruch 5 oder 6, **dadurch gekennzeichnet, daß** mindestens eine elektrisch leitfähige Schicht (31, 32, 33) das Sensorelement käfigartig umgibt.

8. Sensorvorrichtung nach einem der Ansprüche 5 bis 7, **dadurch gekennzeichnet, daß** zumindest eine elektrisch leitfähige Schicht (31, 32, 33) mit einer elektrischen Spannung beaufschlagbar ist.

9. Sensorvorrichtung nach einem der Ansprüche 4 bis 8, **dadurch gekennzeichnet, daß** das Filterelement (23) mehrere elektrisch leitfähige Schichten (31, 32, 33) aufweist, die mit unterschiedlichen elektrischen Spannungen (35, 36, 37) beaufschlagbar sind.

10. Sensorvorrichtung nach einem der voranstehenden Ansprüche, **dadurch gekennzeichnet, daß** das Sensorelement (20) potentialfrei geschaltet werden kann.

11. Sensorvorrichtung nach einem der voranstehenden Ansprüche, **dadurch gekennzeichnet, daß** das Sensorelement (20) eine haftvermindernde Schutzschicht aufweist.

12. Sensorvorrichtung nach einem der voranstehenden Ansprüche, **dadurch gekennzeichnet, daß** die Sensorvorrichtung (50; 80) ein piezoelektrisch in mechanische Schwingungen versetzbares Schwingteil (72) aufweist, das mit dem Sensorelement (70) gekoppelt ist.

13. Sensorvorrichtung nach Anspruch 12, **dadurch gekennzeichnet, daß** das Sensorelement (70) an einer Trägerplatine (71) festgelegt ist, die am Schwingteil (72) gehalten ist.

14. Sensorvorrichtung nach einem der voranstehenden Ansprüche, **dadurch gekennzeichnet, daß** dem Sensorelement (70) ein mechanisches Abstreifelement (75) zugeordnet ist, das relativ zum Sensorelement (70) entlang von dessen sensitiver Oberfläche bewegbar ist.

15. Sensorvorrichtung nach Anspruch 14, **dadurch gekennzeichnet, daß** das Abstreifelement (75) mit einem Aktor (78) gekoppelt ist zum Bewegen des Abstreifelements (75) entlang der sensitiven Oberfläche des Sensorelements (70).
